# EUROPEAN PATENT APPLICATION

(11) **EP 3 617 304 A1**
(43) Date of publication of application: **04.03.2020**
(21) Application number: 18191984.6
(22) Date of filing: 31.08.2018
(51) Int. Cl.: C12M 1/00, C12M 1/34, C12M 1/36

(54) **SYSTEM FOR THE CONTROLLED EXECUTION OF A BIOTRANSFORMATION PROCESS**

(71) Applicant: C-CIT Sensors AG, 8820 Wädenswil (CH)
(72) Inventor: Spichiger, Stefan, 8820 Wädenswil (CH); Caglayan, Ferdi, 8805 Richterswil (CH)
(74) Representative: Pharma Patents International AG

(57) **Abstract**

The present invention relates to a system comprising at least the components
a) a reaction vessel (20) ; b) at least one sensor (30) located at least partly within the inner lumen of the reaction vessel and being in physical contact with the liquid medium and being adapted to monitor at least one parameter related to a cultivation process; c) a measuring and detection unit (40) connected to the at least one sensor;
d) a data transmission unit (50) adapted for the transmission of data be-tween the measuring and detection unit and a processing unit; e) a processing unit adapted for the processing, storage and optionally display of data received from the measuring and detection unit; f) at least one storage vessel (60) adapted for holding further liquid medium, the at least one storage vessel having a fluid connection (80) with the reaction vessel; and g) at least one pump adapted (70) for conveying the further liquid medium contained in the at least one storage vessel to the inner lumen of the reaction vessel.

## Description

### FIELD

The present invention relates to the field of biochemical reactions and transformations, e.g. by cultivation or fermentation of cell cultures or enzymatic transformations in liquid media. More specifically, the present invention relates to a system for the controlled execution of a biotransformation, such as a cultivation or fermentation process of a cell culture or a transformation in the presence of a biocatalyst in a liquid medium. Furthermore, the present invention relates to a process for the controlled execution of biotransformation processes in liquid media.

### BACKGROUND

Biochemical reactions or transformations have become a standard tool for the production or manufacture of a large variety of target compounds ranging from small organic molecules to large biological macromolecules such as carbohydrates or peptides. In many cases, these transformations are implemented by living cell cultures which are often cultivated in liquid media, in most cases aqueous liquid media such as cultivation broths. Usually, cultivation broths comprising living cell cultures are complex and, in most cases, heterogeneous mixtures comprising, i.a. the cell culture, a liquid medium, substrates to be transformed, starting materials, nutritive substances, metabolites resulting from the biochemical transformations, product compounds and more.

In many cases, especially in the context of biotransformation by or in the presence of living cell cultures or biocatalysts, the precise control of the reaction conditions is of outmost importance. Due to the complexity of the reaction mixtures and metabolic transformations involved; however, it is difficult to determine or measure the exact reaction conditions present in the reaction mixture at a specific point in time, which, however, is an indispensable basis for an optimized biotransformation process.

Biotransformation processes involving living cell cultures or biocatalysts are usually conducted in closed reaction vessels, and the status and progress of the reaction or transformation is usually monitored by taking samples of the reaction mixture and subsequent analysis thereof. However, especially in cases in which small to medium-sized volume bioreactors or bioreactor vessels are used, e.g. for screening purposes, taking samples often is a disruptive process that always bears the risk of contamination of the reaction mixture or reduction of the volume left for the continuation or completion of the biotransformation. In these cases, furthermore, disposable bioreactor vessels for single-use are often employed. These small to medium-sized volume bioreactors, however, usually do not permit to take samples from the reaction medium, or to monitor the progress of the biotransformation by other means. Furthermore, these bioreactors are often operated under sterile and controlled environmental conditions, such as in an incubator or other breeding chamber, e.g. with controlled temperature, air humidity and sterility.

Accordingly, it is an object of the present invention to provide a system or other means that allows for the controlled execution of a biotransformation, such as a cultivation or fermentation process in a liquid medium by allowing for optimized control or monitoring of the reaction conditions and progress of said biotransformation process, with minimized disruptive interaction with the liquid reaction medium and/or the living cell culture or the biocatalyst, and by further allowing for an adaption of the reaction conditions in accordance with the results and data gathered by said control or monitoring process. Furthermore, it is an object of the present invention to provide a system that meets the above-mentioned criteria and is suitable for the implementation with disposable, single-use bioreaction vessels as well as for implementation in large number of parallel biotransformations.

Further objects of the present invention will become apparent from the present disclosure including the examples and claims.

### SUMMARY OF THE INVENTION

In the first aspect, the invention relates to a system for the controlled execution of a biotransformation in a liquid medium, the system comprising at least the components:
a) a reaction vessel having an inner lumen for holding the liquid medium;
b) at least one sensor located at least partly within the inner lumen of the reaction vessel and being in physical contact with the liquid medium and being adapted to monitor at least one parameter related to the biotransformation process;
c) a measuring and detection unit connected to the at least one sensor;
d) a data transmission unit adapted for the transmission of data between the measuring and detection unit and a processing unit;
e) a processing unit adapted for the processing, storage and optionally display of data received from the measuring and detection unit;
f) at least one storage vessel adapted for holding further liquid medium, the at least one storage vessel having a fluid connection with the reaction vessel; and
g) at least one pump adapted for conveying the further liquid medium contained in the at least one storage vessel to the inner lumen of the reaction vessel.

In a second aspect, the present invention relates to a regulatory unit for the controlled execution of a biotransformation in a liquid medium, the regulatory unit comprising the components b) to g) of the system according to the first aspect of the invention, and being adapted to be connected to a reaction vessel.

In a third aspect, the present invention relates to a method for the controlled execution of a biotransformation in a liquid medium, characterized by the use of the system according to the first aspect of the invention.

In a fourth aspect, the present invention relates to a computer program comprising instructions to cause the system of the first aspect of the invention, or the regulatory unit of the second aspect of the invention, to execute the steps of the method of the third aspect of the invention.

In a fifth aspect, the present invention relates to a computer-readable medium having stored thereon the computer program of the fourth aspect of the invention.

### BRIEF DESCRIPTION OF THE DRAWINGS

Fig. 1 shows a system for the controlled execution of a biotransformation in a liquid medium according to the present invention with a non-transparent reaction vessel.
Fig. 2 shows a system for the controlled execution of a biotransformation in a liquid medium according to the present invention with a transparent reaction vessel.

### DETAILED DESCRIPTION OF THE INVENTION

The present invention provides a system for the controlled execution of a biotransformation in a liquid medium, the system comprising at least the components:
a) a reaction vessel having an inner lumen for holding the liquid medium;
b) at least one sensor located at least partly within the inner lumen of the reaction vessel and being in physical contact with the liquid medium and being adapted to monitor at least one parameter related to the biotransformation;
c) a measuring and detection unit connected to the at least one sensor;
d) a data transmission unit adapted for the transmission of data between the measuring and detection unit and a processing unit;
e) a processing unit adapted for the processing, storage and optionally display of data received from the measuring and detection unit;
f) at least one storage vessel adapted for holding further liquid medium, the at least one storage vessel having a fluid connection with the reaction vessel; and
g) at least one pump adapted for conveying the further liquid medium contained in the at least one storage vessel to the inner lumen of the reaction vessel.

The system for the controlled execution of a biotransformation in a liquid medium according to the present invention allows for the controlled execution or implementation of a biotransformation or biotransformation process (as used herein synonymously) and comprises or consists of at least the components a) to g) as listed above. The system according to the present invention may, however, additionally comprise one or more further components suitable for the controlled execution of a biotransformation in a liquid medium as will be outlined in more detail below.

In the context of the present invention, the term "biotransformation" as used herein refers to a reaction or cultivation process in the presence of a living or non-living cell culture or biocatalyst by which chemical or biochemical molecules (substrates) are converted into other chemical or biochemical molecules (products). The term "biocatalyst" as used herein refers to any biological molecule suitable for the transformation of chemical or biochemical compounds and having accelerating effect on the transformation. Examples for said biocatalysts comprise enzymes or cell fragments. In specific embodiments, the biotransformation may be, for example, a cultivation process of a cell culture or a transformation in the presence of a biocatalyst.

The term "cultivation process", comprised by the term "biotransformation", as used herein refers to a process of breeding, growing, proliferating or fermenting of a living cell culture. The term "cell culture" as used herein refers to any living biological material being a cell or a clustered group of at least two cells suitable for cultivation in a reaction vessel according to the invention. Examples for biological material that may be cultivated as cell cultures comprise prokaryotes such as e.g. bacteria, eukaryotes, such as e.g. yeasts, fungi, algae, plant or animal cells.

According to component a), the system of the present invention comprises a reaction vessel having an inner lumen for holding the liquid medium, more specifically for holding the liquid medium containing or comprising the cell culture or biocatalyst performing the biotransformation. The reaction vessel can have any form suitable for performing a biotransformation in a liquid medium and/or for holding liquid media typically used for performing a biotransformation or cultivation process. Such liquid media may be aqueous, water-based media, typically composed of a complement of amino acids, vitamins, inorganic salts, glucose, and serum as a source of growth factors, hormones, and other factors. However, the liquid media can also comprise or consist of other suitable factors compatible with the biotransformations to be performed in the context of the present invention, such as factors able to maintain pH and osmolality. The liquid medium further comprises the living or non-living biocatalyst or cell culture performing the biotransformation.

The reaction vessel can be provided in any suitable form or shape known to the skilled person to be suitable for performing cultivation processes; for example, in the form of a flask, a bottle, a tube, a bag or a cylinder; it can be shaped spherical, angular, flat, dish-like, conical, or Erlenmeyer-shaped. It can have a form that allows it to stand or lie, or to be fixed by additional holding means to e.g. a shaker, an incubator or to any other device typically used for performing a cultivation process.

The system according to the invention can be implemented or used independent from the volume of the inner lumen of the reaction vessel. The volume of the inner lumen of the reaction vessel may, for example, range from several millilitres to several litres. The inner lumen of the reaction vessel can, for example, have a volume of about 1 ml to about 10 l, or about 10 mlto about 5 l, or about 100 ml to about 11.

These volumes of the inner lumen of the reaction vessel are chosen such as to hold volumes of the chosen liquid medium in the range of from about 1 ml (often used for screening purposes) up to as large as several litres, such as e.g. up to 5 l, or up to 10 l or up to 20 l (as often used for technical production purposes). In specific embodiments, the volume of the liquid medium ranges from about 1 ml to about 10 l, or from about 10 ml to about 5 l, or from about 100 mlto about 11.

The reaction vessel according to component a) can be made of any material suitable for holding a liquid medium for a biotransformation or cultivation process. The material of the reaction vessel can, for example, be a polymeric material, such as PET, PET-G or polystyrene, glass, metal or any other suitable material known to the person skilled in the art. In specific embodiments, the reaction vessel is made of polymeric material, such as PET, PET-G or polystyrene. In further embodiments, the reaction vessel can be of medical grade polymer, optionally being certified after United States Pharmacopeia (USP) to be a USP class VI polymer.

In a preferred embodiment of the invention, the reaction vessel can be sealed by a closure means adapted for preventing leakage and/or for excluding potential contamination of the liquid medium inside the reaction vessel. The closure means allows for the effective closure of the reaction vessel as well as, in its detached or opened state, for the charging of the reaction vessel, e.g. with the cell culture, the liquid reaction medium or other components or constituents of the liquid reaction medium. In specific embodiments, the closure means can be a cap, or it can be a connecting unit as described in further detail below. In some embodiments, the closure means can seal the reaction vessel, for example, with help of a thread and/or a rubber-seal. In specific embodiments, the closure means can have a vent adapted for continuous gas exchange while maintaining sterility and preventing leakage. In specific embodiments, the closure means can have an additional means for the attachment of the other components of the system, for example the sensor b) or the fluid connection.

In specific embodiments of the invention, a commercially available set containing a reaction vessel and a corresponding, fitting closure means can be used with the system according to the invention for performing a cultivation process. In other embodiments, the closure means can be any available means suitable for the closure of the reaction vessel, that may be provided or purchased separately from the reaction vessel and assessed to be suitable for sealing the reaction vessel during the biotransformation or cultivation process. In specific embodiments of the invention, the closure means is comprised in the connecting unit as described below prior to performing the biotransformation or cultivation process.

In specific embodiments, the reaction vessel of component a) can be a reaction vessel for single-use. The terms "single-use" and "disposable", as used synonymously herein in connection with the reaction vessel or other components of the system of the present invention, mean that the reaction vessel or such other component is used for one biotransformation, cultivation or fermentation process only, and is disposed of or discarded after use, for example, after termination of the biotransformation process and harvesting the cells, other reagents or the generated products from the liquid medium. In preferred embodiments, the reaction vessel being adapted for single-use is sterilisable.

In alternative embodiments, the reaction vessel can also be for multiple-use. The term "multiple-use" as used herein in connection with the reaction vessel or other components of the system of the invention, means that the reaction vessel or such other component can be cleaned after its use, for example, after finishing the biotransformation or cultivation process, and then re-used repeatedly. Cleaning for repeated use can include a sterilisation step, in which case the reaction vessel needs to be sterilisable; i.e. made from a sterilisable material.

In specific embodiments, the reaction vessel can be any commercially available reaction vessel suitable for the implementation of a biotransformation, such as a cultivation, growth or fermentation of living cell cultures or a biotransformation in the presence of an enzyme. Examples of suitable reaction vessels that are commercially available comprise PET-G Erlenmeyer Flasks or Spinner Flasks by Corning; Shaker flasks by Merck; Bio-One™ cell culture roller flasks by Greiner; T-flasks or TubeSpin by TPP; CellSTACK® or HYPERStack® flasks by Corning; WAVE Cellbag by GE-Health Care.

According to component b), the system of the present invention comprises at least one sensor located at least partly within the inner lumen of the reaction vessel and being in physical contact with the liquid medium and being adapted to monitor at least one parameter related to the biotransformation process.

The sensor according to component b) of the invention can be any device, module, or subsystem which can monitor at least one parameter related to the biotransformation or cultivation process. In the context of the present invention, the terms "to monitor/monitoring", "to monitor/monitoring a parameter" or "to monitor/monitoring at least one parameter" mean the detection of events or changes in either the composition of the liquid medium (e.g. its chemical composition) or one or more physical parameters thereof, and the transmission of the information received thereby to the measuring and detection unit according to component c) as described in detail below.

In the context of the present invention, the terms "parameter" or "parameter related to biotransformation process" mean any physical parameter changing or altering during the cultivation process, or a concentration any of chemical compound metabolized, produced and/or necessary in the growing process, or cultivation process.

In specific embodiments of the invention, the at least one parameter related to the biotransformation process; for instance, the 1 to 5 parameters, or 1 to 4 parameters, or 1 to 3 parameters, or 2 to 4 parameters, or 2 parameters, or the just one parameter, is/are selected from physical parameters and concentrations of chemical compounds or constituents in the liquid medium. In specific embodiments, the at least one parameter is selected from the group consisting of glucose concentration, lactate concentration, pH, concentration of ammonium ions, CO₂ and/or O₂ concentration, cell density, volume, and temperature of the liquid medium. More specifically, the at least one parameter is selected from the group consisting of glucose concentration, lactate concentration, pH, and ammonium ion concentration. Further specifically, the at least one parameter is the concentration of glucose and/or lactate in the liquid cell culture medium.

The at least one sensor can be any sensor suitable for monitoring at least one parameter related to the biotransformation process, and specifically for monitoring any of the above-described parameters. Optionally, the at least one sensor can be selected from screen-printed, enzyme-based, electrochemical, or polymer-coated sensors. Suitable sensors according to component b) of the present invention are commercially available; for example, CITSens Bio or CITSens MeMo as provided by C-CIT Sensors AG.

Suitable sensors according to component b) can comprise a recognition element adapted for recognition of a parameter related to the cultivation process to be monitored, optionally a transducing element adapted for converting the signal received by the recognition element and transmitting the signal to a connecting element of the sensor or to the measuring and detection unit according to component c) as outlined below, and a connecting element adapted for linking the transducing element and/or the recognition element to the measuring and detection unit. The recognition element can, for example, be sensitive with regard to molecular, chemical, physical and physicochemical recognition as known to those of skill in the art.

In specific embodiments, the at least one sensor of component b) is connected to the measuring and detection unit of component c), for instance, by a physical connection, optionally by a releasable physical connection. In a more specific embodiment, the sensor can comprise a plug-in element adapted to fit into a recess in the measuring and detection unit for being physically, and optionally releasable, connected. The connecting element of the sensor can, in some specific embodiments, be bent so that the plug-in element can be connected to the measuring and detection unit.

In specific embodiments, the at least one sensor of component b) is adapted for in-situ monitoring. The term "in-situ" refers to the monitoring of at least one parameter as described above on the site where, and at the time when, the events or changes of parameters take place; for example, in the liquid medium without first isolating the liquid medium or a part of the liquid medium characterized by said parameter from the reaction vessel, or without first altering the original conditions in the liquid medium. In the context of the present invention, such "in-situ" monitoring is enabled i.a. by the fact that the at least one sensor is located at least partly within the inner lumen of the reaction vessel and is in physical contact with the liquid medium.

In some embodiments, the sensor is only partly in contact with the liquid medium. More specifically, the elements of the sensor being in physical contact with the liquid medium may be the recognition element, and optionally the transducing element, of the sensor. In some more specific embodiments, a part of the connecting element of the sensor can additionally be in physical contact with the liquid medium.

In some embodiments of the invention, the at least one sensor of component b) is adapted for continuous monitoring of the at least one parameter related to the biotransformation process. In the context of the invention, the terms "continuous/continuously" and "real-time" as used synonymously herein in connection with the monitoring, mean a permanent or non-punctual monitoring of a parameter, such that the obtained signal or information related to a change of said parameter can be transmitted and/or processed directly and continuously immediately after detection of the parameter change, without delay and without interruptions. In a preferred embodiment of the invention, the signal or information is transmitted without delay to the processing unit according to component e) of the present system, as described in further detail below, which is adapted to activate, deactivate, and/or control the at least one pump without a delay or with very minor delay; for instance, with a delay of not more than 1 min, or not more than 30 sec, or not more than 10 sec.

In alternative embodiments of the invention, the at least one sensor according to component b) is adapted for monitoring of the at least one parameter related to the biotransformation process at regular intervals from every about 10 min to every about 1 sec. Preferably, the information is transmitted without delay to the processing unit according to component e) adapted to activate, deactivate, and/or control the at least one pump without a delay or with very minor delay; for instance, with a delay of not more than 1 min, or not more than 30 sec, or not more than 10 sec.

In specific embodiments, the at least one sensor of component b) is adapted to monitor at least 2 different parameters per sensor, for instance, 2 to 10 different parameters per sensor, or 2 to 4 different parameters per sensor, or 2 different parameters per sensor.

In some specific embodiments of the invention, the at least one sensor is adapted to monitor the at least 2 different parameters per sensor simultaneously. The term "simultaneously" as used herein in connection with the monitoring means that at least two monitoring activities, for instance, monitoring of two different parameters, are started or continuously performed in parallel at the same instant.

In some embodiments of the invention, the system comprises 1 to 10 sensors per reaction vessel, or 1 to 5 sensors per reaction vessel, preferably 2 sensors per reaction vessel, most preferably one sensor per reaction vessel. In some specific embodiments of the invention, the multiple sensors are adapted to monitor the at least 2 parameters simultaneously.

In a preferred embodiment of the invention, the system comprises one sensor per reaction vessel, the sensor being adapted to monitor 2 different parameters per sensor. In a more preferred embodiment of the invention the system comprises one sensor per reaction vessel, the sensor being adapted to monitor 2 different parameters per sensor, the parameters being glucose concentration and lactate concentration in the liquid reaction medium.

In specific embodiments, the sensor according to component b) is adapted to monitor the parameters related to the biotransformation or cultivation process in-situ and real-time, as described above. In other specific embodiments, the sensor according to component b) is adapted to monitor the parameters related to the biotransformation or cultivation process in-situ, real-time and simultaneously.

In a preferred embodiment of the invention, the multiple parameters measured by one sensor or by at least two sensors per vessel are monitored in situ, real time and simultaneously.

In some embodiments of the invention, the at least one sensor of component b) is adapted to work without calibration over a period of at least 21 days, more preferably over a period of 35 days. In one embodiment of the invention the at least one sensor is adapted to work accurate after single-point calibration which preferably is necessary after a period of 21 days, more preferably after 35 days.

According to component c), the system of the present invention comprises a measuring and detection unit connected to the at least one sensor. The measuring and detection unit is adapted to receive the signal or information from the at least one sensor according to component b) and to present or transduce the signal or information to the transmission unit.

The measuring and detection unit according to component c) can be physically connected to the at least one sensor, this connection can be a releasable connection, more specifically a plug-in connection. In a more specific embodiment, the measuring and detection unit is connected to the connecting element of the at least one sensor via the plug-in element of the sensor. In specific embodiments, the measuring and detection unit can have a recess to receive the plug-in element of the sensor.

In specific embodiments, the measuring and detection unit c) is adapted to be physically connected or is physically connected to the data transmission unit according to component d) as described in further detail below. In specific embodiments, it may be placed in a housing, optionally in one housing together with the data transmission unit of component d). In some specific embodiments of the invention, the transmission unit c) can be connected to the measuring and detection unit d) by plugging into a recess adapted for receiving the transmission unit.

In specific embodiments, the measuring and detection unit c) may be any electronic device known to the skilled person to be suitable for receiving the signal or information from the at least one sensor according to component b) and to present or transduce the signal or information to the transmission unit.

In some embodiments of the invention, the system further comprises as an additional component a connecting unit adapted to connect the reaction vessel to the components of the system and adapted to close the reaction vessel. In specific embodiments of the invention, the connecting unit is adapted to connect the reaction vessel with the components of the system selected from the components b) to d), f) and g). In a more specific embodiment of the invention, the connecting unit is adapted to connect or physically connects the reaction vessel with the components b) to d), f) and g) of the system. In a more specific embodiment of the invention, the connecting unit is adapted to connect the reaction vessel with components b) to d) and g) of the system and optionally with the fluid connection of the storage vessel according to component f). In alternative embodiments of the invention, however, the connecting unit is adapted to connect the reaction vessel with components b), c) and g) of the system and optionally with component f) of the system or with the fluid connection of the storage vessel according to component f).

The connecting unit can specifically be adapted to connect elements or parts of the above-mentioned components as described above. In preferred embodiments, the components of the present system connected or attached to each other by the connecting element form a single unit. The optional connecting unit further has means for forming a physical connection to the reaction vessel. The connecting unit in these embodiments forms the connection of the components b) to d), g) and optionally f) to the reaction vessel a). In further specific embodiments, the connecting unit forms the connection of the connecting element of the sensor b) or of the housing of the measuring and detection unit c).

In specific embodiments of the invention, the optional connecting unit is a closure means adapted to close the reaction vessel a) as described above. In a more specific embodiment of the invention the connecting unit is a cap adapted to close the reaction vessel.

In further embodiments of the invention, the connecting unit is assembled or preassembled, preferably in one unit, before the biotransformation process is started. The assembly can be done by providing the above-mentioned components of the system which shall be connected to the reaction vessel by the connecting unit, attaching these components to the connecting unit and connecting the connecting unit to the reaction vessel a) containing the cell culture or biocatalyst in the liquid medium, for example, by screwing (e.g. in case of a cap having a thread) or by plugging into the opening of the reaction vessel (e.g. in case of a rubber sealed plug-in connection).

In another embodiment, however, some of the components of the system which shall be connected to the reaction vessel can be mounted to the connecting unit after connecting the connecting unit to the reaction vessel. Specifically, it is conceivable that the sensor of component b) is mounted to the connecting unit before connecting the connecting unit to the reaction vessel and the components selected from c), d), f) and g) are mounted afterwards.

The connecting unit can be adapted such that the components of the system selected from components b) to d), f) and g) are physically attached to said connecting unit. In a specific embodiment, the physical attachment can be a releasable connection. In a preferred embodiment of the invention, the connecting unit comprises means for the permanent or releasable physical attachment of the connected components, such as, for example, recesses, holes or hooks or any other holding means suitable to connect the above-mentioned components to the connecting unit.

In some embodiments of the invention, however, the connecting unit can be adapted such that the components of the system selected from components b) to d), f) and g) are physically attached to said connecting unit in a permanent, non-releasable manner, such as, for example by gluing or permanent fixation by bolts or screws.

In specific embodiments of the invention, the connecting unit and the components selected from b) to d), f) and g) of the system form one unit.

In specific embodiments of the invention, the invention comprises a kit comprised of a connecting unit as described above, a processing unit according to component e) of the system and a reaction vessel according to component a) of the system, the reaction vessel optionally being a single-use, disposable reaction vessel.

According to component d), the system of the present invention comprises a data transmission unit adapted for the transmission of data between the measuring and detection unit and a processing unit. In a preferred embodiment of the invention, the data transmission unit d) is adapted for wireless transmission of the signal, data or information received from the sensor of component b) via the measuring and detection unit c) to the processing unit according to component e) as described in further detail below. More specifically, the transmission unit d) can be adapted to use any possible wireless transmission technology suitable to transfer information between two or more points that are not connected by an electrical conductor. Examples for such wireless transmission technologies comprise, for example, radio waves, Bluetooth communication, near-field communication (NFC), WiFi or other suitable technologies as known to those of skill in the art.

The transmission unit according to component d) of the invention can be any available device suitable for the transmission of data from the measuring and detection unit c) to the processing unit e); such device can be any commercially device suitable for said data transmission as known to the skilled person including wire-based devices or preferably wireless devices. In a preferred embodiment of the invention, the transmission device is a Bluetooth device or a RFID device or any other wireless transmission device known to the skilled person.

In alternative embodiments, the transmission unit can also be adapted for wired transmission of data, using for example a LAN cable connection.

In specific embodiments, the transmission unit can additionally be adapted for the transmission of data between a processing unit according to component e) and a pump according to component g) of the invention; both components are described further below.

According to component e), the system of the present invention comprises a processing unit adapted for the processing, storage and optionally display of data received from the measuring and detection unit. The processing unit according to component e) of the system can be any commercially available data-handling or computing device suitable for the processing, storage and optionally display of data received from the measuring and detection unit; such device can be any commercially device suitable for said data transmission or processing as known to those of skill in the art, such as, for example, personal computers, mobile devices, tablets or smartphones.

In specific embodiments, the processing unit e) comprises an interface adapted for the exchange of information between the measuring and detection unit and the processing unit and/or the for exchange of information between the at least one pump.

In specific embodiments of the invention, the processing unit is adapted or may be programmed to set a predetermined value of at least one of the parameters to be monitored and to automatically activate and/or deactivate the at least one pump according to component g) of the system of the present invention when the parameter reaches the predetermined value. More specifically, the processing unit e) may comprise an input element adapted to receive the predetermined value of at least one of the parameters to be monitored and an output element adapted to send activation and/or inactivation signals to the at least one pump according to component g), the output element optionally being the above-mentioned interface.

In specific embodiments of the invention, the processing unit comprises a software or computer program adapted to compare the actual value of the monitored parameter with the predetermined value of the corresponding parameter to be measured and to automatically activate and/or deactivate or regulate the at least one pump according to component g) when the value of the monitored parameter reaches or approaches the predetermined value of the corresponding parameter.

In some specific embodiments of the invention, the processing unit comprises an interface adapted for the exchange of information between the measuring and detection unit c) and the processing unit e) and/or the for exchange of information between the at least one pump according to component g), an input element adapted to receive the predetermined value of at least one of the parameters, an output element adapted to send activation and/or inactivation or regulation signals to the at least one pump, the output element optionally being the above-mentioned interface and the optional software application or computer program adapted to compare the value of the monitored parameter with the predetermined value of the corresponding parameter and to automatically activate and/or deactivate or to regulate the at least one pump when the value of the monitored parameter reaches the predetermined value of the corresponding parameter.

According to component f) the system of the present invention comprises at least one storage vessel adapted for holding further liquid medium, the at least one storage vessel having a fluid connection with the reaction vessel. In the context of the invention, the storage vessel can be described as being an element or further vessel separate from the reaction vessel and having a fluid connection to the reaction vessel, the fluid connection also being an element separate from the reaction vessel.

In specific embodiments of the invention, the at least one storage vessel may be physically attached to the reaction vessel by additional holding means. In a more specific embodiment, the physical attachment can be a releasable or non-releasable connection. More specifically, the holding means can be ribbons, elastic bands, belts, adhesive tape, glue or other holding means known to the skilled person to be suitable as holding means.

The storage vessel according to component f) of the system can be any available vessel distinct from the reaction vessel according to component a) which is suitable for holding further liquid medium that may be used or necessary for performing, optimizing, extending or somehow regulating the biotransformation or cultivation process conducted in the reaction vessel a) and which is suitable for having a fluid connection with the reaction vessel. The form of the storage vessel f) can be any suitable form, for example, the form of a pouch, a bag, a flask, a bottle, a tube, a cylinder, an Erlenmeyer vessel or it can be club-shaped, spherical, angular, flat, dish-like, conical, or have any other form known to the skilled person being suitable for holding liquid media. It can also have a form that allows to stand or lie, or being fixed by the above-mentioned additional holding means to the reaction vessel according to component a) or to a stand. In preferred embodiments, however, the form of the storage vessel is the form of a pouch, a bag or a flask, more preferably the form of a pouch or a bag, more preferably being attached by holding means to the reaction vessel.

In specific embodiments of the invention, the further liquid medium held by the storage vessel may comprise chemical and/or biological components such as further cell culture or other biocatalyst that are suitable for performing, optimizing, prolonging, attenuating or somehow altering or influencing the biotransformation, cultivation or fermentation processes of the cell culture in the liquid medium or, more specifically, suitable for the controlled execution of such a process.

In specific embodiments, the further liquid medium may comprise, for example, buffer components suitable for establishing a defined pH in the liquid medium and in the further liquid medium, chemical substances or biomolecules suitable to be metabolised by cells in the case of cell cultures or to be transformed, such as glucose, glutamate, amino acids or other components or constituents being of influence for the biotransformation or cultivation process, such as sera, albumins or coenzymes. In specific embodiments of the invention, the further liquid medium held by the storage vessel comprises further cell cultures or biocatalysts, preferably the same cell cultures or biocatalysts as cultivated in the reaction vessel of component a). In specific embodiments of the invention, the further liquid medium held by the storage vessel comprises chemical and/or biological components that are suitable for the adjustment of the parameters of the liquid medium, such as, for example, hydrogen carbonate, dissolved CO₂, dissolved O₂ or phosphate buffer.

The volume of the inner lumen of the storage vessel may, for example, range from several millilitres for liquid media to several litres for liquid media. As described above, the inner lumen of the reaction vessel can, for example, have a volume of about 1 ml to about 10 l, more specifically about 10 ml to about 5 1, or most specifically 100 ml to about 11. In specific embodiments, accordingly, the typical volume of the further liquid medium suitable to be held by the storage vessel may range from about 1 ml to about 10 l, or from about 10 mlto about 5l, or from about 100 ml to about 1l.

The storage vessel according to component f) can be made of any material suitable for holding further liquid medium for a cultivation process. The material of the storage vessel can, for example, be glass, metal or a polymeric material, preferably a medical grade polymeric material such as PET, PET-G or polystyrene, polyethylene, or any other suitable material known to the person skilled in the art. In preferred embodiments, however, the storage vessel is made of polymeric material, such as, for example, PET, PET-G, polystyrene or polyethylene.

In specific embodiments, the at least one storage vessel is sterilisable, preferably by x-ray irradiation. In a more specific embodiment, the at least one storage vessel is a disposable vessel for single-use. In a more specific embodiment, the at least one storage vessel is sterilisable and disposable. In alternative embodiments of the invention, however, the storage vessel can also be for multiple-use. In this case, the storage vessel needs to be sterilisable.

In specific embodiments of the invention, the storage vessel f) can be sealed by a closure means adapted for preventing leakage and/or for excluding potential contamination of the further liquid medium inside the storage vessel. The closure means allows for the effective closure of the storage vessel as well as, in its detached or opened state, for the charging of the storage vessel with further liquid medium, e.g. with the cell culture or other biocatalysts, or other components or constituents suitable for performing the biotransformation process. In specific embodiments, the closure means of the storage vessel can be a cap or it can be a tape, preferably an adhesive tape. In some embodiments, the closure means can seal the storage vessel, for example, with help of a thread and/or a rubber-seal.

In specific embodiments, the at least one storage vessel according to component f) of the system comprises a fluid connection to the reaction vessel according to component a). This fluid connection is adapted to provide a fluid supply between the storage vessel and the reaction vessel, the fluid supply preferably being from the storage vessel f) to the reaction vessel a). Specifically, the fluid connection can be a tube or pipe suitable for supply or transport of fluids, such as the liquid media for the cell cultivation or other biotransformation. The tube or pipe can be made of any material suitable for the supply or the transport of fluid, specifically it can be made of a polymeric material, such as, for example PET, PET-G, polystyrene or polyethylene, more specifically the tube or pipe can be sterilisable.

In further specific embodiments, the fluid connection reaches into the inner lumen of the at least one storage vessel f) the open end of the fluid connection being in physical contact with the further liquid medium. In a more specific embodiment, the fluid connection reaches through the closure means of the storage vessel, the contact region between the closure means of the storage vessel and the fluid connection preferably being effectively closed and preventing contamination of the further liquid medium.

In a specific embodiment, the fluid connection reaches into the reaction vessel a), more specifically, the fluid connection can be in contact with the closure means of the reaction vessel. In a preferred embodiment, the contact region between the closure means of the reaction vessel and the fluid connection is also effectively closed and is adapted such that contamination of the further liquid medium is prevented.

In another specific embodiments of the invention, the fluid connection can be connected to the pump according to component g) as described in detail below, the pump being placed between the at least one storage vessel and the reaction tube.

According to component g) the system of the present invention comprises at least one pump adapted for conveying the further liquid medium contained in the at least one storage vessel to the inner lumen of the reaction vessel. The at least one pump according to component g) of the system can be in fluid connection with the at least one storage vessel of component f) and the reaction vessel of component a), and in wireless or wired, preferably, however, in wireless connection with the processing unit according to component e).

In specific embodiments, the at least one pump g) can be activatable, deactivatable, and/or controllable by the processing unit. In some embodiments, the at least one pump therefore comprises a wireless pump module adapted for receiving signals from the processing unit and for transforming said signals into an activity of the pump. More specifically, the wireless pump module is adapted for transforming said signals into activation, deactivation steps of the pump or into speeding up or slowing down the delivery rate of the pump. In specific embodiments the wireless pump module can be the transmission unit according to component d) of the system.

In specific embodiments, the at least one pump is a micropump adapted to control and manipulate small amounts of liquid or fluid volumes, preferably the liquid medium or the further liquid medium. Micropumps according to the invention can by any commercially available micropump known to the skilled person to be suitable for the purpose of this invention, for example tube pumps or membrane pumps. Said micropumps typically have delivery rates from about 0.2 ml/min to about 1300 ml/min and typically have sizes from about 1 mm to 5 cm side length.

In specific embodiments of this invention, the at least one pump can be any commercially available pump suitable for conveying the further liquid medium contained in the at least one storage vessel to the inner lumen of the reaction vessel. Suitable pump that are commercially available comprise, for example: Mp6-PI Micropump by Bartels Mikrotechnik, P200 Black Edition by Xavitech, BPS-215i by Nitto Kohki, RP-Q1 miniature tube pump by BMT Fluid Control Solutions.

In specific embodiments, the components selected from the group consisting of components a), b), c), d), f), and g) of the system are sterilisable, preferably by x-ray irradiation. In the context of the invention, the sterilisation can be made by any process known to the skilled person to be suitable to sterilise components such as components a), b), c), d), f), and g) of the system. Sterilisable components allow the invention to be used multiple times (multiple use). In a more specific embodiment of the invention, it is also conceivable, that selected components of the above-mentioned list are sterilisable, allowing these components to be used multiple times (multiple use).

In further specific embodiments, the components selected from the group consisting of components a), b), c), d), f), and g) of the system are for single-use or disposable as described above.

In further specific embodiments of the invention, the at least one sensor b), the measuring and detection unit c), the data transmission unit d), the processing unit e), and/or the at least one pump g) are battery driven. More specifically, the battery can be a rechargeable battery. The battery can be any battery or accumulator suitable for providing the above-mentioned components with energy and enable them to perform according to the functions described above.

In other specific embodiments, the system is adapted for parallel use of a plurality of systems. The terms "use in parallel" or "parallel use" herein means the execution of a controlled biotransformation in a liquid medium at the same time or during the same or an overlapping period of time and, preferably in close proximity to each other, e.g. in a shaker or a sterile hood or chamber, such that a plurality of controlled cultivation processes of a cell culture can be executed in each system under essentially the same conditions simultaneously. The parallel use can be carried out with a plurality of systems each comprising the components a) to g) and optionally the connecting unit as described above and/or a plurality of further pumps for the supply of gas as described below.

In specific embodiments, the parallel use of a plurality of systems can also be the executed with a plurality of components a) to d), f) and g), optionally a plurality of connecting units and/or a plurality of further pumps for the supply of air or other gases as described below and one processing unit according to component e), the processing unit being adapted for the processing, storage and optionally display of data received from the plurality of measuring and detection units c) of the plurality of systems according to the present invention. More specifically, the processing unit according to component e) can also be adapted to activate, deactivate, and/or control a plurality of pumps according to component g) of a plurality of systems according to the present invention. In other words, within the scope of the present invention a processing unit according to component e) can be part of a plurality of systems according to the present invention.

In specific embodiments of the invention, the system comprises a further pump or one or more further pumps adapted for the supply of gas, the gas being air and/or another gas such as oxygen, carbon dioxide or a protecting gas like nitrogen or argon, preferably air, and more preferably sterilised air. The at least one further pump according to this embodiment of the invention can be any available pump suitable for the supply of gas to the liquid medium which can be adapted to the system, if necessary with help of other means, for example, a gas connection. Specifically, the further pump can also be a micropump. In some embodiments, the further pump can have the same features as the pump according to component g) of the invention.

In specific embodiments of the invention, the further pump suitable for the supply of gas can be in connection with the processing unit according to component e) and can be adapted to be activated, deactivated and/or controlled or regulated by the processing unit. The connection between the processing unit and the further pump can be a wireless connection.

The system according to the present invention offers, inter alia, the following advantages:
- a reduction of manual labour due to the reduced number of sampling step for the control of the cultivation process;
- a reduced danger of contamination due to the reduced number of contacts of potentially contaminated equipment during sampling;
- a reduced or no influence or interference of the growth of the cell culture, thereby ensuring higher product yield, higher reproducibility od cultivation processes, faster and more precise information on the growth and behaviour of the cells allowing interventions, e.g. providing fresh liquid media, without delay.

In a second aspect, the present invention provides a regulatory unit for the controlled execution of a biotransformation in a liquid medium, the regulatory unit comprising the components b) to g) of the system of the present invention and adapted to be connected to a reaction vessel. The reaction vessel according this aspect of the invention can be any reaction vessel suitable for the cultivation of a cell culture and can have the same features as the reaction vessel according to component a) of the invention. In specific embodiments, the reaction vessel can be a disposable or single-use reaction vessel. Alternatively, it can be a multiple-use reaction vessel. Optionally, the reaction vessel can be sterilisable.

In specific embodiments of the second aspect of the invention, the regulatory unit can comprise a connecting unit adapted to connect a reaction vessel as described above to the components b) to d), f) and g) of the system and adapted to close the reaction vessel. In connection with the regulatory unit according this aspect of the invention, the components b) to g) may be as described above in connection with the system of the present invention, including all specific or preferred embodiments. Furthermore, the connecting unit may have the same features as described above for the system of the first aspect of the invention.

In specific embodiments, the regulatory unit comprises
- at least one sensor according to component b) of the system located at least partly within the inner lumen of the reaction vessel and being in physical contact with the liquid medium and being adapted to monitor at least one parameter related to the biotransformation or cultivation process,
- a measuring and detection unit according to component c) of the system connected to the at least one sensor,
- a data transmission unit according to component d) of the system adapted for the transmission of data between the measuring and detection unit and a processing unit,
- a processing unit according to component e) of the system adapted for the processing, storage and optionally display of data received from the measuring and detection unit,
- at least one storage vessel according to component f) of the system adapted for holding further liquid medium, the at least one storage vessel having a fluid connection with the reaction vessel,
- at least one pump according to component g) of the system adapted for conveying the further liquid medium contained in the at least one storage vessel to the inner lumen of the reaction vessel,
- optionally a connecting unit adapted to connect the reaction vessel to the components b) to d), f) and g) of the system and adapted to close the reaction vessel; and
- optionally a further pump adapted for the supply of gas.

In specific embodiments of the of this second aspect of the invention, the regulatory unit forms one unit, preferably a unit in which the components as defined above are releasably or permanently attached or fixed to each other. In some embodiments, the regulatory unit forms one unit with all above-listed components except for the processing unit according to component e) of the system, the processing unit being in wireless connection with the regulatory unit or with at least one of the above-listed components.

In specific embodiments of the invention, the invention comprises a kit comprised of a regulatory unit as described above and a reaction vessel such as a reaction vessel according to component a) of the system, the reaction vessel optionally being a single-use, disposable reaction vessel.

In a third aspect, the present invention provides a method for the controlled execution of a biotransformation or cultivation process in a liquid medium, characterized by the use of the system according to the first aspect of the invention.

In specific embodiments of this third aspect, the method for the controlled execution of a biotransformation or cultivation in a liquid medium comprises the following steps:
- Providing the system according to the first aspect of the invention;
- Filling the reaction vessel with the liquid medium, the liquid medium optionally comprising the cell culture or the biocatalyst,
- Optionally filling the further liquid medium into the at least one storage vessel in a sterile environment;
- Arranging, connecting and/or assembling the components a) to g) of the system of the first aspect of the invention and/or the regulation unit according to the second aspect of the invention;
- Running the biotransformation or cultivation process, thereby monitoring at least one parameter related to the biotransformation or cultivation process;
- Transmitting data from the measuring and detection unit to the processing unit;
- Comparing said data with a predetermined value of the at least one parameter; and
- Optionally activating, deactivating and/or regulating the at least one pump, thereby supplying further liquid medium to the reaction vessel.

In specific embodiments, the method comprises arranging, connecting and/or assembling the connecting unit and the processing unit of component e) of the system, optionally arranging, connecting and/or assembling other component of the system as described in the first aspect of the invention.

In specific embodiments, the steps of the method as listed above can be carried out in any chronological order suitable for the controlled execution of a biotransformation or cultivation process according to the invention.

In specific embodiments, the steps of the method according to this aspect of the invention as listed above can be repeated as suitable for the controlled execution of a biotransformation or cultivation process according to the invention. More specifically, the following steps can be conducted repeatedly, i.e. a plurality of times, such as twice, 5 times, 10 times or even more as necessary:
- Running the biotransformation or cultivation process, thereby monitoring the at least one parameter related to the biotransformation or cultivation process,
- Transmitting data from the measuring and detection unit to the processing unit,
- Comparing said data with a predetermined value of the at least one parameter, and
- Optionally activating, deactivating and/or regulating the at least one pump, thereby supplying further liquid medium to the reaction vessel.

In specific embodiments, the method further comprises supplying air and/or another gas to the reaction vessel. In specific embodiments, the method comprises activating, deactivating and/or regulating the further pump, thereby supplying air and/or another gas to the reaction vessel.

In specific embodiments of this third aspect of the invention, the method comprises providing the regulatory unit according to the second aspect of the invention and the reaction vessel according to component a) of the system and arranging and/or connecting the regulatory unit and the reaction vessel.

In further specific embodiments, the steps of the method can be carried in an incubator adapted to perform a biotransformation or cultivation process in an environment with defined conditions, for example temperature, CO₂ concentration, pH and/or humidity. The incubator can also provide a sterile environment.

In a fourth aspect, the present invention provides a computer program comprising instructions to cause the system of the first aspect of the invention, or the regulatory unit of the second aspect of the invention, to execute the steps of the method of this third aspect of the invention. In specific embodiments, the computer program comprises instructions to cause the system or the regulatory unit of the present invention to execute the following steps of the method:
- Transmitting data from the measuring and detection unit c) to the processing unit e) via the data transmission unit d);
- Comparing said data with a predetermined value of the at least one parameter; and
- Optionally activating, deactivating and/or regulating the at least one pump, thereby supplying further liquid medium to the reaction vessel.

In specific embodiments of the fourth aspect of the invention, the computer program is stored on the processing unit according to component e) of the system.

In a fifth aspect, the present invention provides a computer-readable medium having stored thereon the computer program of the fourth aspect of the invention.

### DETAILED DESCRIPTION OF THE DRAWINGS

The following is a list of numbered embodiments comprised by the present invention:

| | | | |
|---|---|---|---|
| 10 | System | 50 | Transmission unit |
| 20 | Reaction vessel | 60 | Storage vessel |
| 21 | Closure means of the reaction vessel (20) | 70 | Pump |
| 30 | Sensor | 71 | Wireless pump module of the pump (70) |
| 31 | Recognition element of the sensor (30) | | |
| 32 | Plug-in element of the sensor (30) | 80 | Fluid connection |
| 33 | Connecting element of the sensor (30) | 90 | Connecting unit |
| 40 | Measuring and detection unit | 100 | Processing unit |

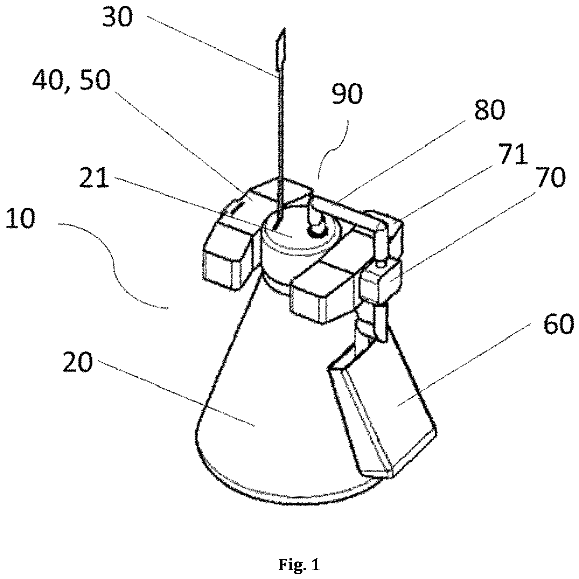

Fig. 1 shows a system (10) for the controlled execution of a biotransformation or cultivation process in a liquid medium according to the present invention with a non-transparent reaction vessel (20). The system (10) as shown in Fig. 1 exemplarily represents the components a) to d), f) and g) of the system (10). The reaction vessel (20) has the form of an Erlenmeyer flask and comprises an interior volume that can be at least partially filled with a liquid medium and the cell culture or the biocatalyst. The reaction vessel (20) is closed by the connecting unit (90) which comprises the closure means (21), being exemplarily a vent cap, the sensor (30), the measuring and detection unit (40) with the transmission unit (50), the wireless pump module (71), the pump (70) and the fluid connection (80).

The sensor (30) is shown in part only reaching through the closure means (21) into the interior of the non-transparent reaction vessel (20). The measuring and detection unit (40) is placed in one housing together with the transmission unit, which is not visible in the Fig. 1. The measuring and detection unit (40) and the transmission unit (50) are attached to the connecting unit (90) via the housing. The wireless pump module (71) and the pump (70) are attached to each other and are attached to the connecting unit (90) via the wireless pump module (71). The fluid connection (80) reaches from the interior of the storage vessel (60) to the pump (70), connects to the ports of the pump (70) thus permitting the liquid medium to flow through the pump (70) when the pump (70) is activated and further, the fluid connection (80) is attached to the connecting unit (90). The storage vessel (60) is attached to the outer wall of the reaction vessel (20) and can contain the further liquid medium.

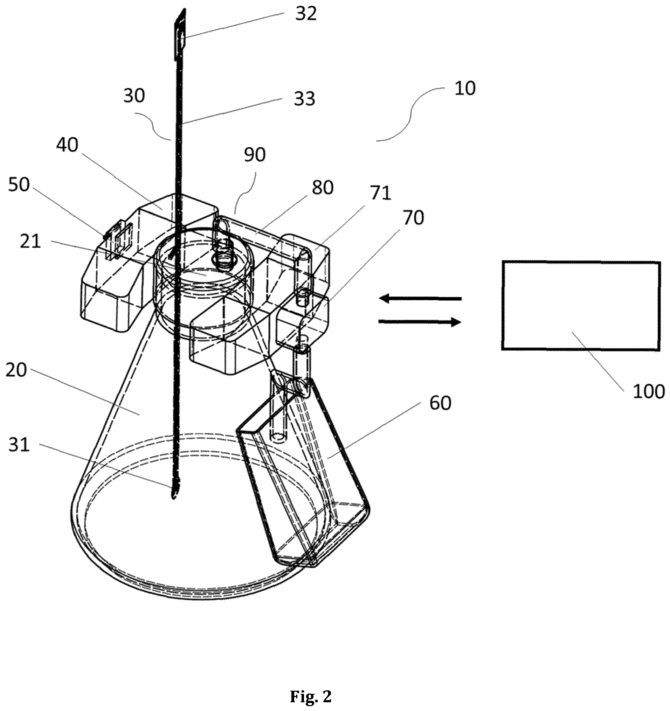

Fig. 2 shows a system for the controlled execution of a biotransformation or cultivation process in a liquid medium according to the present invention with a transparent reaction vessel. The system (10) as shown in Fig. 2 exemplarily represents the components a) to g) of the system (10) and further components comprised by this example. Fig. 2 shows the reaction vessel (20) with a cap (21), the sensor (30) with the recognition element (31), the plug-in element (32) and the connecting element (33), the measuring and detection unit (40), the transmission unit (50), the storage vessel (60), the pump (70) with the wireless pump module (71), the fluid connection (80), the connecting unit (90) and the processing unit (100).

The processing unit (100) is in wireless contact to the other components of the system (10) as marked by the arrows. The processing unit (100) can receive and/or send data from the transmission unit (50) and/or the pump (70) or the wireless pump module (71), represented by the arrows showing in two directions.

The sensor reaches into the lumen of the reaction vessel (20) such that the recognition element (31) can come in physical contact with the liquid medium and is able to monitor at least one parameter related to the cultivation process. The transducing element of the sensor is not shown in Fig. 2 as this is on most cases a thin layer directly attached to or covered by the recognition element. The connecting element (33) and the plug-in element (32) connect the recognition element (31) of the sensor (30) with the measuring and detection unit (40). For that purpose, the closure means (21) of the connecting unit (90) has a recess for the connecting element (33) and the measuring and detection unit (40) has a recess for the plug-in element (32) of the sensor (30). The connecting element (33) of the sensor (30) can be bent so that the plug-in element (32) can be plugged in.

The fluid connection (80) reaches into the lumen of the storage vessel (60) such that the fluid connection (80) can come in physical contact with the further liquid medium and the further liquid medium can be conveyed with help of the pump (70).

The following is a list of numbered items representing embodiments comprised by the present invention:
1. System (10) for the controlled execution of a biotransformation in a liquid medium, the system comprising the components
   a) a reaction vessel (20) having an inner lumen for holding the liquid medium;
   b) at least one sensor (30) located at least partly within the inner lumen of the reaction vessel and being in physical contact with the liquid medium and being adapted to monitor at least one parameter related to the biotransformation process;
   c) a measuring and detection unit (40) connected to the at least one sensor;
   d) a data transmission unit (50) adapted for the transmission of data between the measuring and detection unit and a processing unit;
   e) a processing unit adapted for the processing, storage and optionally display of data received from the measuring and detection unit;
   f) at least one storage vessel (60) adapted for holding further liquid medium, the at least one storage vessel having a fluid connection (80) with the reaction vessel; and
   g) at least one pump (70) adapted for conveying the further liquid medium contained in the at least one storage vessel to the inner lumen of the reaction vessel.
2. System according to item 1, wherein the biotransformation comprises a cultivation process of a cell culture or a transformation in the presence of a biocatalyst.
3. System according to item 1 or 2, wherein the reaction vessel is a single-use reaction vessel.
4. System according to items 1 to 3, wherein the system comprises a connecting unit (90) adapted to connect the reaction vessel to the components of the system and adapted to close the reaction vessel.
5. System according to item 4, wherein the connecting unit is adapted to connect the reaction vessel with components b) to d), f) and g) of the system.
6. System according to item 5, wherein the connecting unit and the components b) to d), f) and g) of the system form one unit.
7. System according to any of items 4 to 6, wherein the connecting unit is a cap adapted to close the reaction vessel.
8. System according to any preceding item, wherein the at least one sensor is adapted for continuous monitoring of at least one parameter related to the biotransformation process.
9. System according to any preceding item, wherein the at least one sensor is adapted to monitor at least 2 different parameters per sensor.
10. System according to any preceding item, wherein the at least one sensor is adapted to monitor 2 to 4 different parameters per sensor.
11. System according to any preceding item comprising one sensor per reaction vessel.
12. System according to any of items 8 to 11, wherein the multiple parameters are monitored simultaneously, in situ and real time.
13. System according to any preceding item, wherein the at least one parameter related to the cultivation process is selected from physical parameters and concentrations of chemical compounds in the liquid medium.
14. System according to any preceding item, wherein the at least one parameter is selected from the group consisting of glucose concentration, lactate concentration, pH, concentration of ammonium ions, CO₂ and/or O₂, cell density, volume, and temperature of the liquid medium.
15. System according to any preceding item, wherein the at least one parameter is selected from the group consisting of glucose concentration, lactate concentration, pH, and ammonium ion concentration.
16. System according to any preceding item, wherein the at least one parameter is the concentration of glucose and/or lactate in the liquid cell culture medium.
17. System according to any preceding item, wherein the at least one sensor is adapted to work without calibration over a period of at least 21 days.
18. The system according to any preceding item, wherein the measuring and detection unit is physically connected to the at least one sensor.
19. The system according to any preceding item, wherein the measuring and detection unit is physically connected to the data transmission unit and/or is placed in one housing with the data transmission unit.
20. The system according to any preceding item, wherein the at least one storage vessel is physically attached to the reaction vessel.
21. The system according to any preceding item, wherein the at least one storage vessel is sterilisable.
22. System according to any preceding item, wherein the at least one storage vessel and/or the reaction vessel is made of glass or a medical grade polymeric material.
23. System according to any preceding item, wherein the components selected from the group consisting of components a), b), c), d), f), and g) are sterilisable, preferably by x-ray irradiation.
24. System according to any preceding item, wherein the components selected from the group consisting of components a), b), c), d), f), and g) are for single-use.
25. System according to any preceding item, wherein the further liquid medium held by the storage vessel comprises further cell cultures.
26. System according to any preceding item, wherein the further liquid medium held by the storage vessel comprises chemical and/or biological components that are suitable for the adjustment of the parameters of the cell culture liquid medium.
27. System according to any preceding item, wherein the processing unit is adapted to set a predetermined value of at least one of the parameters and to automatically activate and/or deactivate the at least one pump when the parameter reaches the predetermined value.
28. System according to item 27, wherein the processing unit is adapted to automatically activate and/or deactivate the at least one pump when the parameter reaches the predetermined value via the transmission unit.
29. System according to any preceding item, wherein the data transmission unit is adapted for wireless transmission of data.
30. System according to any preceding item, wherein the system is adapted for parallel use of a plurality of systems.
31. System according to any preceding item, wherein the at least one pump is in fluid connection with the at least one storage vessel and the reaction vessel, and in wireless connection with the processing unit.
32. System according to any preceding item, wherein the at least one pump is activatable, deactivatable, and/or controllable by the processing unit.
33. System according to any preceding item, wherein the at least one pump is a micropump.
34. System according to any preceding item, wherein the at least one sensor, the measuring and detection unit, the data transmission unit, the processing unit, and/or the at least one pump are battery driven.
35. System according to any preceding item, wherein the system comprises a further pump suitable for the supply of air and/or another gas.
36. System according to item 35, wherein the further pump suitable for the supply of air and/or another gas is in connection with the processing unit and is adapted to be activated, deactivated and/or controlled by the processing unit.
37. Method for the controlled execution of a biotransformation in a liquid medium, characterized by the use of the system according to any of items 1 to 36.
38. Method for the controlled execution of a biotransformation in a liquid medium according to item 37, comprising the following steps:
   - Providing the system according to any of items 1 to 36;
   - Optionally filling the further liquid medium into the at least one storage vessel in a sterile environment;
   - Filling the reaction vessel with the cell culture in the liquid medium or the biocatalyst in the liquid medium;
   - Arranging and/or connecting the components a) to g) of the system according to claim 1 and/or the connecting unit according to item 4 to 7;
   - Running the biotransformation process, thereby monitoring at least one parameter related to the biotransformation process and
   - Transmitting data from the measuring and detection unit to the processing unit;
   - Comparing said data with a predetermined value of the parameter;
   - Optionally activating, deactivating and/or regulating the at least one pump, thereby supplying further liquid medium to the reaction vessel.
39. Method for the controlled execution of a biotransformation in a liquid medium according to item 38, wherein the method comprises supplying air and/or another gas to the reaction vessel.

The following example serve to illustrate the present invention without, however, limiting it in any respect:

### EXAMPLES

### Example 1: Cultivation of ChoMaster®EPO cells

A 250 ml Erlenmeyer Shake flask with vented cap (Corning Cat. No. 431145) is equipped with a sensor for the continuous measurement glucose and lactate concentrations (CITSens Bio or CITSens MeMo; C-CIT Sensors AG, Switzerland), a tubular fluid connection and a storage vessel and micropump mp6 (Bartels Mikrotechnik, Germany). The equipped flask is then sterilized by gamma radiation.

The equipped flask is being charged under a clean bench with 10 ml ChoMaster® HP-1 medium (CHP1, Cell Culture Technologies, Switzerland) containing glucose in a concentration of 3 g/l and ChoMaster®EPO cells (Cell Culture Technologies, Switzerland) in a concentration of 5x10⁵ cells/ml. The storage vessel is charged with 100 ml ChoMaster® HP-1 medium containing glucose in a concentration of 3 g/l. The sensor is connected to the measuring and detection unit; the pump is connected to the wireless pump module.

The flask equipped with storage vessel, micropump, sensor, wireless pump module and measuring and detection unit are placed in a shaking incubator (Lab-Therm LT-X, Kühner, Germany) and the incubator is set to 37°C, shaking speed is set to 120 rpm and CO₂ gas enrichment is set to 5%. The cultivation process starts directly after placement of the flask into the shaker.

With opening a new data file in the program stored in the processing unit referring to the running cultivation process directly afterwards, the glucose and lactate concentrations are monitored and saved by the processing unit. The monitored glucose and lactate concentrations are represented by the program in the processing unit and can be seen on a display or printed out.

During the cultivation process it is observed that the glucose concentration is decreasing while the lactate concentration is increasing. This indicates the growth of the cell culture. A predetermined value of a glucose concentration of 1 g/l is set in the processing unit. The pump is automatically started by the processing unit to pump ChoMaster® HP-1 medium containing glucose in a concentration of 3 g/l from the storage vessel until the preset glucose concentration is reached in the liquid medium. After 7 days the cultivation process is terminated by harvesting the cells.

## Claims

1. System (10) for the controlled execution of a biotransformation in a liquid medium, the system comprising the components
a) a reaction vessel (20) having an inner lumen for holding the liquid medium;
b) at least one sensor (30) located at least partly within the inner lumen of the reaction vessel and being in physical contact with the liquid medium and being adapted to monitor at least one parameter related to the biotransformation;
c) a measuring and detection unit (40) connected to the at least one sensor;
d) a data transmission unit (50) adapted for the transmission of data between the measuring and detection unit and a processing unit;
e) a processing unit adapted for the processing, storage and optionally display of data received from the measuring and detection unit;
f) at least one storage vessel (60) adapted for holding further liquid medium, the at least one storage vessel having a fluid connection (80) with the reaction vessel; and
g) at least one pump (70) adapted for conveying the further liquid medium contained in the at least one storage vessel to the inner lumen of the reaction vessel.

2. System according to claim 1, wherein the biotransformation comprises a cultivation process of a cell culture or a transformation in the presence of a biocatalyst in the liquid medium.

3. System according to claim 1 or 2, wherein the reaction vessel is a single-use reaction vessel.

4. System according to any of the claims 1 to 3, wherein the system comprises a connecting unit (90) adapted to connect the reaction vessel to the components of the system and adapted to close the reaction vessel.

5. System according to claim 4, wherein the connecting unit is adapted to connect the reaction vessel with components b) to d), f) and g) of the system.

6. System according to any preceding claim, wherein the at least one sensor is adapted for continuous monitoring of at least one parameter related to the biotransformation.

7. System according to any preceding claim, wherein the at least one sensor is adapted to monitor at least 2 different parameters per sensor.

8. System according to any preceding claim, wherein the multiple parameters are monitored simultaneously, in situ and real time.

9. System according to any preceding claim, wherein the at least one parameter is the concentration of glucose and/or lactate in the liquid medium.

10. The system according to any preceding claim, wherein the at least one storage vessel is physically attached to the reaction vessel.

11. System according to any preceding claim, wherein the components selected from the group consisting of components a), b), c), d), f), and g) are sterilisable.

12. System according to any preceding claim, wherein the components selected from the group consisting of components a), b), c), d), f), and g) are for single-use.

13. System according to any preceding claim, wherein the system comprises a further pump suitable for the supply of air and/or another gas.

14. Method for the controlled execution of a biotransformation in a liquid medium, **characterized by** the use of the system according to any of claims 1 to 13.

15. Method for the controlled execution of a biotransformation in a liquid medium according to claim 14, comprising the following steps:
- Providing the system according to any of claims 1 to 13;
- Filling the reaction vessel with the liquid medium;
- Optionally filling the further liquid medium into the at least one storage vessel in a sterile environment;
- Arranging, connecting or assembling the components a) to g) of the system according to claim 1 to 13 and/or the connecting unit according to claim 3 or 4;
- Running the biotransformation process, thereby monitoring at least one parameter related to the biotransformation;
- Transmitting data from the measuring and detection unit to the processing unit;
- Comparing said data with a predetermined value of the parameter; and
- Optionally activating, deactivating and/or regulating the at least one pump, thereby supplying further liquid medium to the reaction vessel.
